# EUROPEAN PATENT APPLICATION

(11) **EP 3 714 946 A1**
(43) Date of publication of application: **30.09.2020**
(21) Application number: 19166234.5
(22) Date of filing: 29.03.2019
(51) Int. Cl.: A61Q 5/02, A61K 8/60, A61K 8/86, A61Q 19/10, A61K 8/85, C08B 11/04, C08G 63/66, C08K 5/00

(54) **TOPICAL PREPARATIONS COMPRISING POLYALKOXYLATED POLYOLS POLYESTER WITH GUERBET ACID**

(71) Applicant: Applechem Inc., Parsippany-Troy Hills, NJ 07054 (US)
(72) Inventor: LIN, Samuel Q., Paramus, NJ New Jersey 07652 (US); QIN, Xu, Quincy, MA Massachusetts 02170 (US)
(74) Representative: Wichmann, Hendrik

(57) **Abstract**

The subject application relates to topical preparations comprising polyalkoxylated polyols polyester with Guerbet acid. Compounds contained herein relate to polyalkoxylated polyol polyesters having a viscosity that allows a product to be poured, yet retained on a desired surface to which it is applied. Embodiments of the compounds may be exemplified by the formula:

Q-[(OA)ₙ-OR]ₘ

## Description

### FIELD OF THE EMBODIMENTS

The field of the present invention and its embodiments relate to polyalkoxylated polyols polyester that generate a viscosity that allows the product to be poured, yet retained on a desired surface to which it is applied.

### BACKGROUND OF THE EMBODIMENTS

Rheology, or the study of the flow of matter, is applied to consumer products, such as shower gel, shampoo, liquid detergent, dishwashing detergent, hand soap, skin care lotion or cream, hair conditioner, hair styling products, etc. to create a particular viscosity profile. Such a profile is critical to a consumer's product preference and eventual purchasing decision.

Consumers will prefer a product with a rheology profile that causes the product to be stable in the container, have a low enough viscosity to pour out of the container easily, and yet be thick enough to apply to the body, hair, or fabric without dripping out of the consumer's hand or the applied surface. Additionally, the product must be stable and maintain a consistent rheology profile during storage in the warehouse, while in transportation, and while on the shelf for potentially many years.

There are a number of commercial thickeners using esters of polyalkoxylated polyols and fatty acids to thicken surfactant-containing preparations. Examples of commercial products include polyethylene glycol 6000 distearate, also known with INCI name of PEG-150 distearate; PEG 120 methyl glucose dioleate and PEG 120 methylglucose trioleate (Glucomate™ DOE 120 and Glucomate™ VLT); PEG-150 Pentaerythrityl Tetrastearate (Crothix™, CrothixTM Liquid, and Versathix™); PEG-150 Polyglyceryl-2 Tristerate (Genapol LT); PEG/PPG-120/10-Trimethlolpropane Trioleate (Arlypon TT). The number of hydrophilic polyalkoxylated arms is two for PEG-150 distearate, three for Arlypon TT, four for Genapol LT and Crothix, Crothix Liquid, and Versathix, and five for Glucomate DOE 120.

US5,192,462 (Gloor et al.) pertains to a thickening agent comprising a tetra ester made of fatty acids and a polyoxyethylene pentaerythritol with four hydrophilic poly-(ethylene glycol) arms. Its preferred chemical structure is the PEG-150 Pentaerythrityl Tetrastearate, where all four hydrophilic poly-(ethylene glycol) arms are capped with stearic fatty acids, and is the base of Crothix, Crothix liquid, and Versathix Liquid.

US7, 709,011 and US7, 553,495 (both Klug, et al.), pertains to a thickening agent of oxyalkylated polyglycerol esters with fatty acid for surfactant-containing topical preparation as shown below.

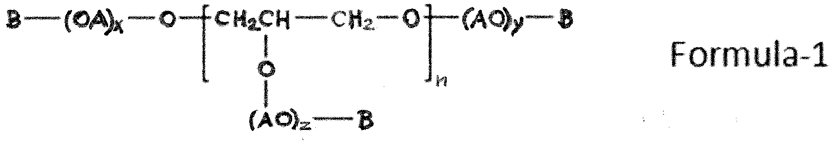

In which A is a group of the formula -C₂H₄- or C₃H₆-, B is a hydrogen or group of the formula -COR, where at least one symbol B is a group of the formula -COR-, R is C₇-C₃₄ - Alkyl, C₇-C₂₁-hydroxyalkyl or Alkenyl, n is a number of 1 to 30, and x, y, z are numbers from 0 to 100, where the sum of x, y, and z is 50 - 250. (*See* Column 1, lines 43 - 48).

To those skilled in the art, the number of hydrophilic poly-(ethylene glycol) - arms equals to n+2. When n =30 and x+y+z = 250, the average number of ethylene glycol units or (x+y+z)/ (n+2) is [250/ (30+2)] and is about 7.81 at maximum.

### SUMMARY OF THE EMBODIMENTS

The embodiments of the present invention teach new polyalkoxylated polyols polyester conforming to the Formula-2 (below), which is a reaction product of (a) fatty acid and (b) polyalkoxylated polyols.

Q-[(OA)ₙ-OR]ₘ Formula-2

wherein Q is a radical of natural or synthetic organic polyols compounds having from 6 to 50 carbon atoms forming a straight, a branched, a cyclic, a saturated, or an unsaturated structure, with each carbon atom being independently substituted with 6 to 25 groups having the formula - [(OA)ₙ-OR], wherein each of the 6 to 50 carbon atoms may independently be substituted with hydrogen, oxygen, or nitrogen; A is selected from -C₂H₄- or -C₃H₆-; R is independently selected from hydrogen or -COR₁, wherein R₁ is independently selected from C₆-C₃₄ alkyl, C₆-C₂₂-hydroxyalkyl, C₂ - C₂₂ alkenyl, and is preferably a stearic moiety, an isostearic moiety, an oleic moiety, a branched Guerbet acid moiety of C₁₆ - C₃₂ or mixtures thereof and the most preferable embodiment has either oleic acid, or alternatively a mixture of isotearic acid and Guerbet acid; the average total number of COR₁ (based on two or more, preferably a multitude, of polyalkoxylated polyols polyester conforming to the Formula-2) is ≥ 2.5, preferably ≥3, and most preferably ≥4; n is an integer selected from 1-125 and may be the same or different for each polyalkoxylated hydrophilic arm; the average number of n per (OA)ₙ unit is from 25 to 120, preferably from 30 to 85, and most preferably from 30 to 70; and m is an integer selected from 6- 25, preferably 6-12.
In one embodiment, the present invention refers to the following items:
1. A polyalkoxylated polyols polyester having the formula:

   Q-[(OA)ₙ-OR]ₘ Formula-2

   Q is a radical of natural or synthetic organic polyol compounds having from 6 to 50 carbon atoms forming a straight, a branched, a cyclic, a saturated, or an unsaturated structure, with one or more (preferably all) carbon atoms independently having a substituent of the formula -[(OA)ₙ-OR], wherein n = 6-25, wherein the remaining carbon atoms of the 6 to 50 carbon atoms are independently substituted with hydrogen, oxygen (preferably -OH), or nitrogen (preferably -NH₂), particularly preferred hydrogen;
   A is selected from -C₂H₄- or -C₃H₆-;
   R is independently selected from hydrogen or -COR₁,
   R₁ is independently selected from C₆-C₃₄-alkyl, preferably derived from a Guerbet acid; C₆-C₂₂-hydroxyalkyl, preferably derived from a Guerbet acid; or C₆ - C₃₄ - alkenyl, preferably derived from a Guerbet acid,
   n is an integer selected from 1-125 and may be the same or different for each polyalkoxylated hydrophilic arm; and the average total number of COR₁ is ≥ 2.5, preferably ≥ 3, and most preferably ≥ 4; and
   m is an integer selected from 6-25, and preferably from 6-12.
2. The polyalkoxylated polyols polyester of item 1, wherein the C₆-C₃₄-alkyl derived from a Guerbet acid has the following formula: 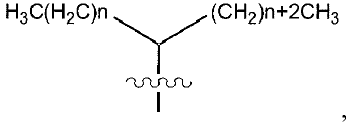 wherein n = 1-120, preferably 3, 5, 7, 9, or 13.
3. The polyalkoxylated polyols polyester of item 1 or 2, wherein n per hydrophilic poly-(alkylene glycol) arm (-[(OA)ₙ-OR]) is equal to 25 to 120, more preferably 30 to 85, and most preferably 30 to 70.
4. The polyalkoxylated polyols polyester of any preceding item, wherein R₁ is derived from stearic, isostearic, oleic, Guerbet acid, or mixtures thereof.
5. The polyalkoxylated polyols polyester of any preceding item, wherein R₁ comprises Guerbet and isotearic moieties.
6. The polyalkoxylated polyols polyester of any preceding item, wherein the Guerbet moiety has 18 to 24 carbon atoms.
7. The polyalkoxylated polyols polyester of any preceding item, wherein Q is the radical of the following polyols compounds:
   Sugar alcohols, having the general formula of HOCH₂(CHOH)ₓCH₂OH;
   Disaccharide, having a glycosidic linkage;
   (C₆H₁₀O₅)ₙ₀, where n0 is from 2 to 20;
   Di-Pentaerythritol;
   Dendrimer polyols; and
   Polyglyceryls having 3 to 10 glycerin units, with 6 or more hydroxyl groups.
8. The polyalkoxylated polyols polyester of any preceding item, wherein Q is selected from the polyols of the following compounds:
   Sugar alcohols, having the general formula of HOCH₂(CHOH)ₓCH₂OH;
   Disaccharide, having a glycosidic linkage;
   (C₆H₁₀O₅)ₙ₀, where n0 is from 2 to 20;
   Di-Pentaerythritol; and
   Dendrimer polyols.
9. The polyalkoxylated polyols polyester of any preceding item, wherein Q is selected from the polyols of sorbitol, trehalose, mannitol, Di-pentaerythritol, sucrose, lactose, chitobiose, cellobiose, and maltose.
10. A cosmetic, dermatological, or pharmaceutical composition comprising the polyalkoxylated polyols polyester of any of the preceding items or mixtures thereof.
11. The cosmetic, dermatological, or pharmaceutical composition of item 10, wherein Q is a radical of sorbitol of the following: 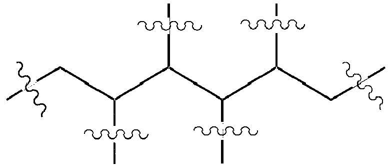
12. The cosmetic, dermatological, and pharmaceutical composition of item 10 or 11, further comprising:
   water;
   1% - 50% by weight of surfactants selected from the groups consisting of anionic surfactant, cationic surfactant, non-ionic surfactant, amphoteric surfactant, and mixtures thereof;
   0.1% to 10% by weight of the polyols polyester of Formula-2; and
   0.1 to 50% by weight of other ingredients selected from the groups consisting of: skin and hair benefit actives, stabilizer, further thickeners, colorants, preservatives, and pearlizing agents.
13. The cosmetic, dermatological, and pharmaceutical composition of item 10, further comprising:
   water;
   2% - 50% by weight of surfactants selected from the groups consisting of: sulfate-free anionic surfactants, non-ionic surfactant, amphoteric surfactant, and mixtures thereof;
   0.1% to 10% by weight of the polyalkoxylated polyols polyester of Formula-2; and
   0.1% to 50% by weight of other ingredients selected from the group consisting of: skin and hair benefit actives, stabilizer, further thickeners, colorants, and preservatives.
14. The cosmetic, dermatological, and pharmaceutical composition of any of items 10-13, further comprising:
   water;
   about 1% to 30% by weight of a skin and hair active ingredient selected from the group consisting of: UV filters, moisturizers, conditioners, antiseptic agents, deodorant actives, reducing agents for permanent wave products, colorants for coloring hair, anti-aging actives, proteins/protein derivatives, perfume, petrolatum, vegetable oils, cationic conditioning polymers, and mixtures thereof; and
   about 1% to 10% by weight of the polyalkoxylated polyols polyester of Formula-1

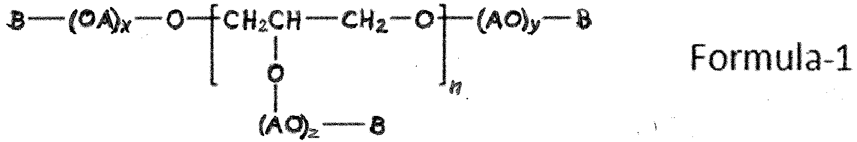

In another embodiment of the invention there are cosmetic, dermatological, and pharmaceutical preparations containing the polyalkoxylated polyols polyester of Formula-2.

The polyester of the embodiments of the invention are suitable as a thickener, rheology modifier, dissolver, conditioner, and dispersant for aqueous, aqueous/alcoholic, and surfactant-containing preparations, and as emulsifiers and suspending agents with a thickening action and a bodying action for emulsions and suspensions. Examples of these surfactant-containing preparations, emulsions, and suspensions are shampoo, shower preparations, shower gels, foam baths, facial cleanser, hand soap, bar soap, shaving creams, hair conditioners, deodorants, lotions, creams, ointments, wet wipes, antiperspirants, sunscreens, etc. The embodiments of the invention are also suitable as a thickener and rheology modifier for fabric care products, such as fabric conditioner and liquid laundry detergent. The invention thus also relates to compositions comprising or consisting of two or more, preferably a multitude, of polyalkoxylated polyols polyester molecules of Formula-2 as described herein, wherein the average total number of COR₁ is defined on the basis of all polyalkoxylated polyols polyester molecules of Formula-2 which are contained in the composition.

Based on the finished formulation, the preparation, the cleansing preparation, the emulsions and suspensions according to this invention comprise preferably 0.05% to 20% by weight, particularly preferably 0.1% to 10% by weight, especially preferably 0.5% to 5% by weight of the polyalkoxylated polyols polyester of Formula-1.

The cleansing compositions according to the embodiments of the present invention can further comprise at least one of the following ingredients: all customary anionic, cationic, zwitterionic, nonionic, and amphoteric surfactants; all customary skin and hair benefit actives such as, for example, cosmetic oils, petrolatum, vegetable oils, hydrogenated vegetable oils, UV filters, proteins, shining agents, anti-aging agents, amino acids, bioactives, humectants, conditioning polymers, silicones, cationic polymers, sucrose polyesters, anti-dandruff zinc salt, hydroxyacids, skin lightening agents; all customary stabilizers, such as, for example, silica, 12-hydroxystearic acid, hydrogenated castor oil, ethylene glycol distearate, bentonite and hectorite clay, fatty acids, fatty alcohols; all customary thickeners such as, for example, hydroxyethyl cellulose, xanthan gum, polyacrylate, modified or non-modified starch, etc; all customary dye, coloring agents, pearlizers, perfumes, chelators, solvents, humectants, salts, etc.

The total amount of the surfactants used in the composition of this invention can, based on the finished composition, be between 5% and 70% by weight, preferably between 10 and 40% by weight, and most preferably between 12% and 35% by weight.

### Definitions

As used above, and throughout this disclosure, the following terms, unless otherwise indicated, shall be understood to have the following meanings. If a definition is missing, the conventional definition known to one skilled in the art controls.

As used herein, the terms "including," "containing," and "comprising" are used in their open, non-limiting sense.

As used herein, the singular forms "a," "an," and "the" include plural referents unless the context clearly dictates otherwise.

To provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value. Whenever a yield or amount is given as a percentage, such yield or amount refers to a mass of the entity (percent by weight) for which the yield is given with respect to the maximum amount of the same entity that could be obtained under the particular stoichiometric conditions. Concentrations that are given as percentages refer to mass ratios, unless indicated differently.

As used herein, "alkyl" means a straight chain or branched saturated chain having from 1 to 34 or more carbon atoms. An alkyl group can be unsubstituted or substituted. Alkyl groups containing three or more carbon atoms may be straight, branched, or cyclized.

As used herein, an "alkenyl" includes an unbranched or branched hydrocarbon chain having one or more double bonds therein and having from 1 to 34or more carbon atoms. The double bond of an alkenyl group can be unconjugated or conjugated to another unsaturated group. An alkenyl group can be unsubstituted or substituted.

The term "hydroxyl" means an OH group;
The term "hydroxyalkyl" means an alkyl group as defined above, where the alkyl group has an OH group disposed thereon.

The term "alkoxy" or "alkoxylated" as used herein includes -O-(alkyl), wherein alkyl is defined above.

As used herein, the term "composition" is intended to encompass a product comprising the specified ingredients in the specified amounts, as well as any product which results, directly or indirectly, from combination of the specified ingredients in the specified amounts.

The term "amino" as used herein means a substituent containing at least one nitrogen atom.

As used herein, the term "substituted" means that the specified group or moiety bears one or more suitable substituents wherein the substituents may connect to the specified group or moiety at one or more positions.

As used herein, the term "unsubstituted" means that the specified group bears no substituents.

Any atom that is represented herein with an unsatisfied or unspecified valence is assumed to have the sufficient number of hydrogen atoms to satisfy the atom's valence. For example, "substituted with oxygen" or "substituted with nitrogen" means that the substituent is oxygen bonded via one bond with one hydrogen (-OH) or via two bonds without hydrogen (=O) and nitrogen bonded via one bond with two hydrogens (-NH2) or bonded via two bonds with one hydrogen (=NH).

All patents or patent applications referenced herein are fully incorporated by reference in their entirety.

### DETAILED DESCRIPTION OF THE EMBODIMENTS OF THE INVENTION

In one embodiment, there is the polyalkoxylated polyols polyester of Formula-2, wherein Q is defined as a radical of natural or synthetic organic polyols compounds, wherein the radical structure is derived from the polyol by removal of one or more, preferably all of, the respective OH-groups, having carbon, hydrogen, oxygen, and nitrogen elements, and being straight, branched, cyclic, saturated, or unsaturated, and wherein Q has from 6 to 50 carbon atoms, and is independently substituted with from 6 to 25 groups having the formula of - [(OA)ₙ-OR]

Q-[(OA)ₙ-OR]ₘ Formula-2

Wherein A is selected from -C₂H₄- or -C₃H₆-; n is from 1-125; R is independently selected from hydrogen or -COR₁; and R₁ is independently selected from C₆-C₃₄-alkyl, C₆-C₂₂-hydroxyalkyl, C₂ - C₃₄ - alkenyl; wherein R₁ is preferably a stearic moiety, an isostearic moiety, an oleic moiety, a Guerbet moiety having 12 to 32 carbon atoms, or mixtures thereof. "-[(OA)ₙ-OR]" is a radical which is attached to the Q radical. A highly preferred R₁ is an oleic moiety; another highly preferred embodiment is a mixture of isotearic acid, and Guerbet acid. In some embodiments, the Guerbet acid has 12 to 32 carbons, more preferably about16-32 carbons, more preferably 18-24 carbons, and most preferably 20 carbons. and the average total number of COR₁ is ≥ 2.5, preferably ≥ 3, and most preferably ≥ 4; and m is an integer selected from 6-25, preferably from 6-12. The n for each hydrophilic poly-(alkylene glycol) - arms can range from 1 - 125, and the average number of n is from 25 to 120, preferably from 30 - 85, and most preferably from 30 to 70.

Guerbet acid is a primary carboxylic acid with well-defined twin branching of carbon chain (formula-3). This unique branching structure results in lower melting point, lower viscosity, and better solubility. Examples of Commercial products are ISOCARB® from Sasol, ranging from 12 carbons to 32 carbons. A preferred range of carbons is 16 to 32 carbons, most preferred is 18 to 24 carbons, with the most preferred number of carbons being 20. 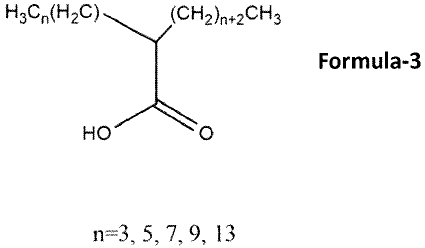 A branched Guerbet acid derived moiety has the following formula: 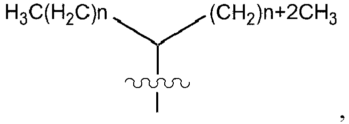 wherein n = 1-120. The symbol " 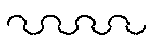 " shows the attachment site of the moiety to the carbon atom of -COR₁. In other words "derived from a Guerbet acid" means that R₁ (alkyl, alkenyl or hydroxyalkyl), together with -COOH, forms a Guerbet acid. The polyalkoxylated polyol polyester (Formula-2) of this invention is prepared by one or more reaction stages. The polyalkoxylated polyols are prepared by the alkoxylation of the polyol compounds, having 6 to 20 hydroxyl groups, with ethylene oxide or propylene oxide at 130°C - 200°C after drying the mixture of the polyols compound and a base catalyst such as KOH, NaOH, or Calcium metal at 100°C -200°C under vacuum. The alkylene oxides are metered into the reactor under pressure over the course of 10-20 hours. Ethylene oxide, propylene oxide, or a mixture of ethylene oxide and propylene oxide can be used, resulting in primary -OH group, secondary -OH group, or mixtures thereof.

The organic polyol compounds (Q) of the embodiments of this invention can be natural polyols or synthetic polyols of having at least six hydroxyl groups, and their examples are shown (but not limited to) from the following classes of compounds:
1. Sugar alcohols, also called polyhydric alcohol or polyalcohol with at least six hydroxyl groups, having the general formula of HOCH₂(CHOH)ₓCH₂OH. Examples include, but not limited to, mannitol, sorbitol, galactitol, fucitol, iditol, inositol, volemitol, isomalt, maltitol, lactitol, maltotritol, maltotetraitol, and polyglycitol.
2. Disaccharide, which is formed from two monosaccharide by dehydration via glycosidic linkage. Examples include but not limited to, trehalose, sucrose, lactose, maltose, etc. They contain six or more hydroxyl groups.
3. Dextrin with a chemical structure of (C₆H₁₀O₅)ₙ, where n is from 2 to 20.
4. Di-Pentaerythritol
5. Dendrimer polyols. For examples, Bolton®H2004, H2003, and H20 have 6, 12, and 16 terminal hydroxyls, respectively.
6. Polyglyceryls with 3 to 10 glycerin units, with six or more hydroxyl groups.
After the reaction, each hydroxyl group of the starting polyol compound will grow to a hydrophilic poly-(alkylene glycol) arm. The length of all arms may be the same or different, depending on the reaction conditions.

The next reaction is an esterification reaction between the polyalkoxylated polyols compounds and the fatty acid, so that some or all of the hydrophilic poly-(alkylene glycol) arms are capped with fatty acid ester. The reaction is carried out between 120°C - 250°C with or without the catalyst until the desired acid number or the degree of esterification is achieved. The preferred method is to use the esterification catalysts such as alkylbenzenesulfonic acid, methansulfonic acid, organotin catalyst, organotitanate catalyst, etc. The preferred mole ratio of fatty acid to the alkoxylated polyols compounds is to form esters of 30% - 100% of the hydrophilic poly-(alkylene glycol) arms. For example, when the starting polyol compound is sorbitol which will lead to six poly-(alkylene glycol) arms per sorbitol molecule, the mole ratio would be 2.4 to 6. When it is trehalose, which will lead to eight poly-(alkylene glycol) arms, the mole ratio is 3.2 to 8. The most preferred one is to form more than at least four fatty ester capped arms.

In another embodiment, Q is the organic radical part of Sorbitol with 6 carbons, and the Formula-4 is the chemical structure of polyols ester from the sorbitol starting compound.

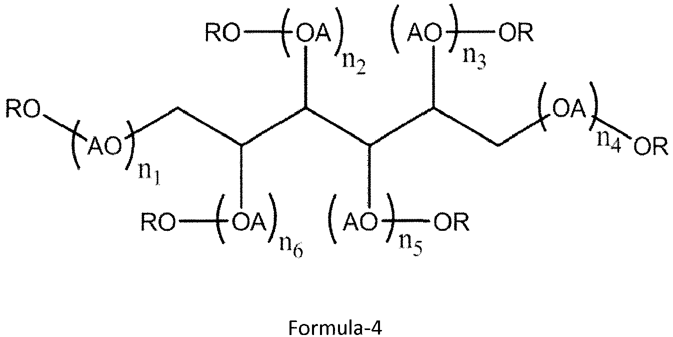

Another embodiment of this invention is the cosmetic, dermatological, and pharmaceutical preparations containing the polyalkoxylated polyols polyester (Formula-1) of this invention. The polyalkoxylated polyols polyester of this invention are suitable as thickener, rheology modifier, dissolver, conditioner, and dispersants for aqueous, aqueous/alcoholic, and surfactant-containing preparations; as emulsifiers and suspending agents with a thickening action and bodying action for emulsions and suspensions. These surfactant-containing preparations, emulsions, and suspensions are, for example, shampoo, shower preparations, shower gels, foam baths, facial cleansers, hand soaps, bar soaps, shaving creams, hair conditioners, deodorants, lotions, creams, ointments, wet wipes, antiperspirants, sunscreens, etc.

Based on the finished formulation, the cleansing preparation, the preparations of emulsions and suspensions according to embodiments of this invention comprise preferably 0.05% to 20% by weight, particularly preferably 0.1% to 10% by weight, especially preferably 0.5% to 5% by weight of the polyalkoxylated polyols polyester of Formula-2 or Formula-3.

The cleansing compositions according to embodiment of this invention can further comprise the following components: all customary anionic, cationic, zwitterionic, nonionic, and amphoteric surfactants; skin and hair benefit actives such as, for example, cosmetic oils, petrolatum, vegetable oils, hydrogenated vegetable oils, UV filters, proteins, shining agent, anti-aging agents, amino acids, bioactives, humectants, conditioning polymers, silicones, cationic polymers, sucrose polyester, anti-dandruff zinc salt, hydroxyacids, skin lightening agents; stabilizers, such as, for example, silica, 12-hydroxystearic acid, hydrogenated castor oil, ethylene glycol distearate, bentonite and hectorite clay, fatty acid, fatty alcohol, etc.; other thickeners such as, for example, hydroxyethyl cellulose, xanthan gum, polyacrylate, modified or non-modified starch, etc.; and polyethylene glycols. The cleansing compositions can be in the forms of liquid, paste, gels, or solid.

The total amount of the surfactants used in the composition of embodiments of this invention can, based on the finished composition, be between 5% and 70% by weight, preferably between 10% and 40% by weight, and most preferably between 12% and 35%.

Each of these components as well as preferred and optional components in the cleansing compositions is described below.

### A. Detersive surfactants

The customary detersive surfactants may be selected from anionic, cationic, non-ionic, amphoteric/zwitterionic surfactants, or mixtures thereof. The details of these customary detersive surfactants are known, such as US 7,659,235 B2; US 8,361,450 B2; US 8,802,607B2; US 3,929,678; US 2,528,378 which are incorporated herein by reference.; and McCutcheon's, Emulsifiers and Detergents, 1989 Annual, published by M.C. Publishing Co. Anionic surfactants may include alkyl sulfate or alkyl ether sulfate (including alkyl glycerol ether sulfate). They may also include the sulfate-free anionic surfactants as illustrated below.

Aliphatic sulfonate, such as a primary alkane (e.g. C₈-C₂₂) sulfonate, primary alkane disulfonate, C₈-C₂₂ alkene sulfonate, alkyl glyceryl ether sulfonate, aromatic alkyl sulfonate, or C₈-C₂₂ hydroxyalkane sulfonate.

Alkyl sulfosuccinates (including mono- and dialkyl, e.g. C₆-C₂₂ sulfosuccinates). Alkyl and acyl taurate, alkyl and acyl glycinates, alkyl sulfoacetate, alkyl phosphates, alkyl phosphate ester, alkyoxy alkyl phosphate esters, and acyl lactates, C₈-C₂₂ monoalkyl succinates and maleate. Fatty acyl isethionates, which are typically prepared by the reaction of an isethionates salts such as alkali metal isethionates and an aliphatic fatty acids of 8-20 carbon atoms. Commercial products of fatty acyl isethionates, for examples, are DEFI, Hostapon SCI-78C, Jordapon CI prill, YA-SCI-85, Iselux LQ-CLR-SB, etc. Anionic carboxylate surfactants with the formula of R-(CH₂CH₂O)ₙCO₂M, wherein R is C₈-C₂₀ alkyl; n is 1 to 20; and M is a positive ion(s) such as sodium, potassium, etc. Another class of anionic surfactants is soap or the salts of fatty acids. Sulfonate derivatives of alkyl polyglucoside, include for example, sodium laurylglucosides hydroxypropylsulfonate, and sodium decylglucosides hydroxypropylsulfonate.

Another sulfate-free mild surfactant is the class of alkanoyl surfactants prepared from the amino acids. The alkyl group is C₈ to C₂₀, preferably C₁₂ to C₁₆ alkyl group. This class of surfactants may include, for examples, alkanoyl sarcosinates, alkanoyl glycinate, and alkanoyl glutamate. The commercial products, for examples, are Amisoft®, Amilite® of Ajinamoto, Eversoft of Sino Lion, etc.

The preferred anionic surfactants are the sulfate-free mild surfactants and their mixtures thereof. The proportion by weight of the anionic surfactants in the composition according to embodiments of this invention is in the range of 5% to 35% by weight, preferably 10% to 25% by weight.

Amphoteric or zwitterionic surfactants are the surfactants with both of positive and negative charges. They can be broadly described as derivatives of aliphatic quaternary ammonium, phosphonium, and sulfonium compounds, in which the aliphatic radicals can be straight or branched chain, and wherein one of the aliphatic radicals is from C₈ to C₁₈ carbon atoms, and one contains an anionic group, e.g., carboxyl, sulfonate, sulfate, phosphate, or phosphonate. Examples may include the customary betaines, such as N-alkyl-N, N-dimethyl ammonium glycinates, coco-amidopropyl betaine; C₁₂-C₁₈-alkyldimethyl-sulfopropylbetain, and amine oxides. The proportion of the amphoteric surfactants in the composition according to embodiments of this invention is 0.5% to 30% by weight, and preferably 1% - 15% by weight.

The non-ionic surfactants may include the alkoxylated aliphatic alcohol, acids, amides or alkyl phenol; long chain tertiary amine oxide; long chain tertiary phosphine oxides; dialkyl sulphoxides; sugar amides, such as described in US 5,389,279 and US 5,009,814; alkyl polyglucoside as described in US 4,565,647 and US 3,723,325, all of which are incorporated herein by reference. The preferred non-ionic surfactants are alkyl polyglucoside and alkyl polyglucamide. Examples of commercial products may include Plantaren® series of BASF, Ecosense ®Series of Dow Chemicals, Gluco Tain® of Clariant, and Poly Suga Mulse of Colonial Chemicals. The proportion of the non-ionic surfactants according to embodiments of this invention is in the range of 1% to 20% by weight, and preferably of 1% to 15%.

Cationic surfactants are the surfactants with positive charge groups. The suitable cationic surfactants may include quaternary ammonium salts, such as di-(C₁₀-C₂₄)-alkyldimethylammonium chloride, (C₁₀-C₂₄)-alkyltrimethylammonium chloride or sulfate, and N-acylaminoethyl-N, N-diethyl-N-methyl ammonium chloride. Other customary cationic surfactants are described in reference of US 8,470,305 B2 and 8,470,305 B2 which are incorporated herein by reference. The proportion by weight of cationic surfactants in the composition according to embodiments of this invention is in the range of 1% to 10%, and preferably 1% to 7% by weight.

### B. Liquid crystal inducers and modifiers

Liquid crystal inducers are small non-ionic molecules and believed to be solubilized in the mixtures of surfactants, to thereby change the packing of surfactant micelles to larger structure aggregates of different shape and size, such as lamellar liquid structures or vesicles, rod and cubic liquid crystals. The alternative name for the liquid crystal inducers is hydrophobic thickeners. They include the class of alkanoamides, alkylamineoxides or mixtures thereof. Examples of this class include mono- and di-ethanolamides, isopropanolamides of fatty acids of 10-20 carbon atoms, PPG-hydroxyethyl cocamides and alkylamineoxides of 10-20 carbon atoms. Another class of chemicals in the liquid crystal inducers is alkoxylated alkyl alcohols of 8-18 carbon atoms, preferably 8-12 carbon atoms and 1-4 ethylene oxide units.

Liquid crystal modifiers include fatty acid and fatty alcohol of 8-20 carbon atom, and aliphatic hydrocarbons of less than molecular weight of 400 g/mole. It is believed that they modify the size and shape of the liquid crystals. US 7,655,607B2 is a reference for the range and examples of the liquid crystal inducers and modifiers, which is incorporated herein by reference.

### C. Skin and hair benefit actives

These benefit actives may be water-soluble, water-insoluble, or water dispersible. The water-soluble actives may include, but not limited to, polyols such as glycerin, diglycerine, sorbitol, propylene glycol, propanediol, panthenol, and sugar; alpha-hydroxyl acids and its salts; low molecular weight polyethylene glycols. Water-insoluble and water-dispersible skin and hair benefit actives include, but not limited to, petrolatum, silicones, vegetable oils, essential oils, emollients, hydrocarbon oils, fatty esters, cationic polymers, oils of high refractive index for shinning, anti-dandruff agents, proteins/protein derivatives, etc. These non water-soluble benefit agents normally exist as emulsion or stripes in the composition. Non-limiting examples in US 7,262,158 are incorporated here by reference. Other miscellaneous skin and hair benefit actives may include vitamins, lipids (sucrose esters, lanoline, cholesterol, etc.), liposome, essential fatty acids, butters, minerals, anti-microbial, anti-acne, oil control agents, astringents, oil control-agents, scrub and exfoliating particles, essential oils, sunscreens, styling aid, dye, perfume, cyclodextrin/perfume complex, anti-wrinkle actives (amino acids and their derivatives such as N-acetyl-L-cystein), thiols, anti-cellulite agents (caffeine, theophylline, etc.), tanning actives, skin lightening actives, skin soothing agents (such as bisabolol, aloe vera, dipotassium glycyrrhizinate, etc.).

Cationic water soluble/or dispersible polymers are very useful for the compositions according to this invention as conditioning actives or deposition aids. The suitable cationic polymers for the compositions according to this invention have the cationic charge density in the range of 0.2 - 8 meq/g and the molecular weight range of 1,000 to 3 million. Their cationic groups are nitrogen-containing moieties such as quaternary ammonium or cationic protonated amino moieties which can be primary, secondary, and tertiary amines. Non-limiting examples of the cationic polymers are described in the CTFA Cosmetic Ingredient Dictionary, 3rd edition, edited by Estrin, Crosley, and Haynes, and US 8470,305 B2, and US 8,105,994 B2 which are incorporated herein by reference. Non-limiting examples may include copolymers of vinyl monomers having cationic protonated amines or quaternary ammonium functionalities with water-soluble spacer monomers such as acrylamide, methacrylamide, alkyl and dialkyl acrylamides, vinyl pyrrolidone, vinyl caprolactone, etc. Non-limiting specific examples are Polyquaternium-11, -16, -7, -6, -22, -47, -39. Other suitable cationic polymers include polysaccharide polymers such as cationic cellulose derivatives, cationic starch derivatives, cationic guar gum derivatives, etc. Non-limiting examples include the following trade name products: Jaguar® from Rhone Poulenc, Aqua® and N-Hance polymer from Aqualon, UCARE Polymer from Dow Chemical, MerQuat from Nalco, Galactasol from Henkel, etc.

### D. Stabilizers and further thickeners

The stabilizers (or structuring systems) are used to form a crystalline stabilizing network in the composition, preventing the droplets of the lipophilic benefit agents from coalescing and phase separation in the product. Non-limiting examples include a hydroxyl-containing fatty acids, fatty ester, or fatty soap water-insoluble wax-like substance such as 12-hydroxystearic acid, 9,10-dihydroxystearic acid, tri-9,10-dihydroxystearin, and tri-12-hydroxystearin. Other class of stabilizers is the C₁₀-C₂₂ ethylene glycol fatty acid ester, fumed silica, precipitated silica, smectite clay, etc. Other customary stabilizer examples are disclosed in US 6,194,363 and US 9,138,428 which are incorporated herein by reference. Another class of stabilizer is a gel-network of fatty amphiphiles such as stearic acid and behenyltrimethylammonium chloride, as disclosed in US 8,470,305. Another class of stabilizer is a blend of non-modified and modified starch and fatty acid as disclosed in US 6,906,016 all of which are incorporated herein by reference..

Further thickeners for stabilizing the composition and for modifying the viscosity of the composition, according to this invention, are polymers. Non-limiting examples include carbohydrate gums such as cellulose gum, microcrystalline cellulose, cellulose gel, hydroxyethyl cellulose, hydroxypropyl cellulose, sodium carboxymethylcellulose, methyl/propyl cellulose, guar gum, gum karaya, gum tragacanth, gum arabic, gum acacia, gum agar, xanthan gum, and mixtures thereof; modified and non-modified starch granules with gelatinization temperature between 30°C - 85°C, and pregelatinized cold water soluble starch. Further non-limiting examples include the class of hydrophobic associative, cross-linked, alkali swellable acrylate polymers, comprising acidic monomers and associative monomers having hydrophobic end groups, as disclosed in US 9,161,899 which is incorporated herein by reference.. Non-limiting commercial examples are Carbopol Aqua SF-1 of Lubrizol, Stabylen 30 of 3V Sigma S.P.A. Aqupec series of Sumitomo Seika of Japan.

Suitable further thickeners may include salt such as sodium chloride and sodium sulfate; cellulose derivatives, such as hydroethylcellulose; xanthan gum, guar gum; starch and starch derivatives; carboxyvinyl polymers, such as Carbopol® 940; Polyacrylate emulsions, such as Carbopol®Aqua SF-1 polymer; polyethylene glycol; and polyvinyl alcohol.

The preparation of the emulsions and suspension, according this invention, comprises water; oils; emulsifiers; preferably 0.05% to 20% by weight, particularly preferably 0.1% to 10% by weight, especially preferably 0.5% to 5% by weight of the polyester of Formula-1; and other customary ingredients for skin care, hair care, and body care. The non-aqueous parts of the emulsion ranges normally from 2% to 85%, and preferably from 5% to 45%. The oils include, but not limited to, cosmetic oils of refined vegetable oils, refined synthetic or fermented hydrocarbon oils, silicone oils, and synthetic ester oils.

The following non-limiting examples demonstrate the composition and the outstanding performances of this invention.

### Examples

**Example 1.** Synthesis of polyalkoxylated polyols polyester of Formula-3 with different molecular structures. Table 1 listed the preparation of Formula-3 polyesters with a range of molecular weight of alkoxylated sorbitol and fatty acids. The reaction flask used is a one liter resin kettle with four necks. The sorbitol was mixed with KOH or NaOH base catalyst, and dried at 90°C -110°C under vacuum. X moles of ethylene oxide or blend of ethylene oxide and propylene oxide per mole of sorbitol are added under pressure and reacted at 140°C - 180°C over the course of 10-20 hours reaction time. After the reaction, the resulting product is cooled down, degassed, and filtered to obtain Sorbeth-xxx. The ingredients of Sorbeth-xxx, oleic acid or stearic acid, and the catalyst - methanesulfonic acid were added into the flask, followed by purging with nitrogen gas. The mixture was heated to between 120°C - 220°C while mixing under the nitrogen and collecting the water. The reaction continued until the sum of R₁ (or acid value) reached the target or near constant. The products were collected after cooling the flak down to room temperatures. The resulting polyesters were waxy solid.

| Table 1. Synthesis of Formula-1 Polyesters | | | | | | | | | |
|---|---|---|---|---|---|---|---|---|---|
| | **Polyester-1** | **Polyester-2** | **Polyester-3** | **Polyester-4** | **Polyester-5** | **Polyester-6** | **Polyester-7** | **Polyester-8** | **Polyester-9** |
| Sorbeth-145 | 76.4% | | | | | | | | |
| Sorbeth-EO/PO 150/10 | | 81.3% | | | | | | | |
| Sorbeth-160 | | | 80.5% | | | | | | |
| Sorbeth-170 | | | | 81.4% | | | | | |
| Sorbeth-220 | | | | | 84.9% | 87% | 86.9% | | |
| Sorbeth-300 | | | | | | | | 90.8% | 88.1% |
| Isostearic acid | | | 19.1% | | | | | | |
| Oleic acid | 23.2% | 18.3% | | | 14.7% | | | | |
| Stearic Acid | | | | 18.2% | | 12.6% | 12.7% | 8.8% | 11.5% |
| 70% methanesulf onic acid | 0.4% | 0.4% | 0.4% | 0.4% | 0.4% | 0.4% | 0.4% | 0.4% | 0.4% |

**Example 2.** The polyester-6 of Table 1 of this invention and PEG-150 Pentaerythrityl Teterstearate were formulated in a non-sulfate personal cleansing composition of 6% of Potassium Cocoyl glycinate (Amilite GCK-1 IF from Ajinomoto Inc), 15% Cocamidopropyl Betaine (35% active by weight, trade name - Monteric LMAB), 0.3% EDTA, 78.7% water, and citric acid to pH 6. The potassium cocoyl glycinate surfactant is derived from the glycine amino acid, and is known to be very mild to the skin. The trade name of PEG-150 Pentaerythrityl tetrastearate is CROTHIX by Croda Inc, and is based on US 5,192,462 which is incorporated herein by reference.
At 2% by weight of thickeners, the Polyester-6 of this invention resulted in a viscosity of 72,900 cP whereas the CROTHIX resulted in a viscosity of 45,840 cP. This shows the superior performance of the polyesters of this invention over the prior art.

**Example 3.** This personal cleansing formulation is based on another amino acid derived surfactant - sodium cocoyl sarcosinate. Its composition is 29.89% of sodium cocoyl sarcosinate ( Trade name - Protelan LS 9011 by Zschimmer & Schwarz, 29% by weight of active), 12.38% of cocamidopropyl betaine (Trade name - Monateric LMAB, 35% by weight of active), 1.8% of thickener, 1% of cocamide MEA, 0.1% EDTA, 1% NaCl, 53.83% of water, and citric acid to pH 6. At 1.8% by weight in the formulation, Polyester-7 of this invention resulted in 6960 cP viscosity whereas CROTHIX resulted in 3640 cP.

**Example 4.** This personal cleansing formulation is based on the non-ionic alkyl polyglucoside surfactant and the anionic sodium cocoyl isethionate, two of well-known mild and non-sulfate surfactants in the personal cleansing market. The composition is 13% Decyl glucoside (Trade name - Plantaren 2000 N UP by BASF), 7% of sodium cocoyl isethionate (Trade name - Pureact I-78C), 7% of cocamidopropyl betaine (Trade name - Montaric LMAB), 1% PEG-7 glyceryl cocoate (Trade name - Protachem GC-7 by Protameen Inc), 0.35% EDTA, citric acid to pH 5.5, and water. At the same 1.2% by weight, the Polyester-4 of this invention and CROTHIX thickened the above surfactant formulation to 26340 cp and 22200 cP, respectively. The polyester 4 of this invention showed a superior performance over the CROTHIX of the prior art again.

**Example 5.** This formulation is the classical sulfate surfactant cleansing products: 10.7% of sodium lauryl ether sulfate solution (70% active by weight), 8.58% of Cocamidopropyl betaine ( 35% active by weight), 0.25% Cocamide MEA, 0.2% EDTA, 0.5% NaCl, x% of thickener, and 79.57% of water. Its pH is 5.5. The polyester-5 of this invention in Table 1 was compared with three commercial thickeners in this sulfate cleansing formulation: 1) Trade name: CROTHIX, solid form, supplied by Croda. INCI: PEG-150 Pentaerythrityl Tetrastearate; 2) Trade name: Glucamate DOE-120, supplied by Lubizol Inc. INCI name: PEG-120 Methyl Glucose Dioleate; 3) Trade name: Rewopal PEG 6000 DS, supplied by Evonik Inc. INCI name: PEG-150 Distearate.

| **Table 2. Thickening Performance Comparison in sulfate surfactant product** | | | |
|---|---|---|---|
| **% concentration of thickener** | **0.2%** | **0.4%** | **0.6%** |
| Polyester-5 | 16580 cP | 37960 cP | 56720 cP |
| CROTHIX | 9760 cP | 21840 cP | 35280 cP |
| Glucamate DOE120 | 500 cP | 600 cP | 700 cP |
| Rewopal PEG 600 DS | 500 cP | 1560 cP | 3040 cP |

This result demonstrated the superior performance of the thickeners of the embodiments of this invention over many of the commercial thickeners of the prior art in the classical sulfate surfactant personal cleansing products. This and other examples demonstrated that a wide range of the polyesters of embodiments of this invention were more effective and versatile thickeners than the current commercial products of prior art in a wide range of personal cleansing products with different type of surfactants.

**Example 6.** This example illustrates the synthesis of Formula-1 polyester with the twin-branched Guerbet acid. The commercial Guerbet acids used were Isocarb-20 (2-octyl-dodecanoic acid), having 20 carbons, and Isocarb-24 (2-decyl-tetradecanoic acid), having 24 carbons, supplied by Sasol. The same reaction condition of example 1 were applied here.

| Table 3. Synthesis of Formula-1 Polyester with Guerbet Acid | | | |
|---|---|---|---|
| | PE-10 | PE-11 | PE - 12 |
| Sorbeth-230 | 82.50% | 82.68% | 82.68% |
| Isocarb-20 | 16.85% | | 6.04% |
| Isocarb-24 | | 6.04% | |
| Oleic acid | | 10.94% | 10.94% |
| Methane Sulfonic Acid, 70% | 0.65% | 0.65% | 0.65% |

**Example 7.** This example demonstrates the thickening power of polyester 11 (PE-11) in two popular cleansing formulations. SH-1 cleansing formulation (shampoo): water 78.9%, potassium cocoyl glycinate 6%, cocamidopropyl betaine 15%, EDTA 0.1%, pH 7; SH-2 cleansing formulation(also shampoo): water 58.4%, sodium C14-16 olefin sulfonate (39% solid) 30%, cocamidopropyl betaine 11.5%, EDTA 0.1%, pH 7. The polyester-11 of this invention showed a much powerful thickening performance than Crothix-S.

| Table 4. Thickening Performance of Polyester of This Invention | | | | | | |
|---|---|---|---|---|---|---|
| | SH1-C | SH1-PE-11 | | SH2-C | SH2-PE-11 | SH2-PE-5 |
| SH-1 | 98% | 98% | | | | |
| SH-2 | | | | 98.3% | 98.3% | 98.3% |
| Corthix-S | 2% | | | 1.7% | | |
| Polyester-11 | | 2% | | | 1.7% | |
| Polyster-5 | | | | | | 1.7% |
| Viscosity, cP | 26,980 | 38,490 | | 1767 | 13,960 | 11,433 |

In addition to showing a strong improvement over the shampoo formulations having Corthix, the formulation with a mixture of Guerbet acid also showed significant improvements over the formulation without Guerbet Acid.

## Claims

1. A polyalkoxylated polyols polyester having the formula:
Q-[(OA)ₙ-OR]ₘ Formula-2
Q is a radical of natural or synthetic organic polyol compounds having from 6 to 50 carbon atoms forming a straight, a branched, a cyclic, a saturated, or an unsaturated structure, with one or more carbon atoms independently having a substituent of the formula -[(OA)ₙ-OR], wherein n = 6-25,
wherein the remaining carbon atoms of the 6 to 50 carbon atoms are independently substituted with hydrogen, oxygen, or nitrogen;
A is selected from -C₂H₄- or -C₃H₆-;
R is independently selected from hydrogen or -COR₁,
R₁ is independently selected from C₆-C₃₄-alkyl, preferably derived from a Guerbet acid; C₆-C₂₂-hydroxyalkyl, preferably derived from a Guerbet acid; or C₆ - C₃₄ - alkenyl, preferably derived from a Guerbet acid,
n is an integer selected from 1-125 and may be the same or different for each polyalkoxylated hydrophilic arm; and the average total number of COR₁ is ≥ 2.5, preferably ≥ 3, and most preferably ≥ 4; and
m is an integer selected from 6-25, and preferably from 6-12.

2. The polyalkoxylated polyols polyester of claim 1, wherein the C₆-C₃₄-alkyl derived from a Guerbet acid has the following formula: 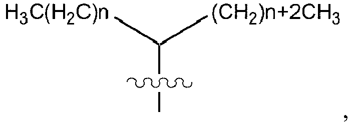 wherein n = 1-120, preferably 3, 5, 7, 9, or 13.

3. The polyalkoxylated polyols polyester of claim 1 or 2, wherein n per hydrophilic poly-(alkylene glycol) arm (-[(OA)ₙ-OR]) is equal to 25 to 120, more preferably 30 to 85, and most preferably 30 to 70.

4. The polyalkoxylated polyols polyester of claim 1 or 2, wherein R₁ is derived from stearic, isostearic, oleic, Guerbet acid, or mixtures thereof.

5. The polyalkoxylated polyols polyester of claim 1 or 2, wherein R₁ comprises Guerbet and isostearic moieties.

6. The polyalkoxylated polyols polyester of claim 1 or 2, wherein the Guerbet moiety has 18 to 24 carbon atoms.

7. The polyalkoxylated polyols polyester of claim 1 or 2, wherein Q is the radical of the following polyols compounds:
Sugar alcohols, having the general formula of HOCH₂(CHOH)ₓCH₂OH;
Disaccharide, having a glycosidic linkage;
(C₆H₁₀O₅)ₙ₀, where n0 is from 2 to 20;
Di-Pentaerythritol;
Dendrimer polyols; and
Polyglyceryls having 3 to 10 glycerin units, with 6 or more hydroxyl groups.

8. The polyalkoxylated polyols polyester of claim 1 or 2, wherein Q is selected from the polyols of the following compounds:
Sugar alcohols, having the general formula of HOCH₂(CHOH)ₓCH₂OH;
Disaccharide, having a glycosidic linkage;
(C₆H₁₀O₅)ₙ₀, where n0 is from 2 to 20;
Di-Pentaerythritol; and
Dendrimer polyols.

9. The polyalkoxylated polyols polyester of claim 1 or 2, wherein Q is selected from the polyols of sorbitol, trehalose, mannitol, Di-pentaerythritol, sucrose, lactose, chitobiose, cellobiose, and maltose.

10. A cosmetic, dermatological, or pharmaceutical composition comprising the polyalkoxylated polyols polyester of any of the preceding claims or mixtures thereof.

11. The cosmetic, dermatological, or pharmaceutical composition of claim 10, wherein Q is a radical of sorbitol of the following:

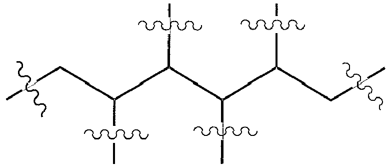

12. The cosmetic, dermatological, or pharmaceutical composition of claim 10 or 11, further comprising:
water;
1% - 50% by weight of surfactants selected from the groups consisting of anionic surfactant, cationic surfactant, non-ionic surfactant, amphoteric surfactant, and mixtures thereof;
0.1% to 10% by weight of the polyols polyester of Formula-2; and
0.1 to 50% by weight of other ingredients selected from the groups consisting of: skin and hair benefit actives, stabilizer, further thickeners, colorants, preservatives, and pearlizing agents.

13. The cosmetic, dermatological, or pharmaceutical composition of claim 10, further comprising:
water;
2% - 50% by weight of surfactants selected from the groups consisting of: sulfate-free anionic surfactants, non-ionic surfactant, amphoteric surfactant, and mixtures thereof;
0.1% to 10% by weight of the polyalkoxylated polyols polyester of Formula-2; and
0.1% to 50% by weight of other ingredients selected from the group consisting of: skin and hair benefit actives, stabilizer, further thickeners, colorants, and preservatives.

14. The cosmetic, dermatological, or pharmaceutical composition of any of claims 10, 11, and 13, further comprising:
water;
about 1% to 30% by weight of a skin and hair active ingredient selected from the group consisting of: UV filters, moisturizers, conditioners, antiseptic agents, deodorant actives, reducing agents for permanent wave products, colorants for coloring hair, anti-aging actives, proteins/protein derivatives, perfume, petrolatum, vegetable oils, cationic conditioning polymers, and mixtures thereof; and
about 1% to 10% by weight of the polyalkoxylated polyols polyester of Formula-1
